# EUROPÄISCHE PATENTSCHRIFT

(11) **EP 0 508 477 B1**
(45) Veröffentlichungstag und Bekanntmachung des Hinweises auf die Patenterteilung: **10.07.1996**
(21) Anmeldenummer: 92106292.3
(22) Anmeldetag: 10.04.1992
(51) Int. Cl.: A61F 13/15

(54) **Wegwerfwindeln**
Disposable diapers
Couches à jeter

(30) Priorität: 10.04.1991 JP 32167/91
(43) Veröffentlichungstag der Anmeldung: 14.10.1992
(73) Patentinhaber: UNI-CHARM CORPORATION, Kawanoe-shi Ehime-ken (JP)
(72) Erfinder: Igaue, Takamitsu, Kawanoe-shi, Ehime-ken (JP); Tanji, Hiroyuki, Kawanoe-shi, Ehime-ken (JP)
(74) Vertreter: Sperling, Rüdiger, Dipl.-Ing.

(56) Entgegenhaltungen:
- EP-A- 0 376 022
- EP-A- 0 421 473
- GB-A- 2 216 393

## Beschreibung

Die Erfindung betrifft Wegwerfwindeln zum Absorbieren und Aufnehmen von Exkrementen.

In den japanischen amtlichen Mitteilungen zur Offenlegung von Patentanmeldungen Nr. 1986-41304 ist eine Wegwerfwindel aufgezeigt, bei der ein dem Schrittbereich entsprechender Teil einer oberen Lage ausgeschnitten ist und so eine längliche Öffnung bildet, die zur Aufnahme von Exkrementen dient, und an der elastische Elemente in nicht umhüllter Form an der Unterseite der oberen Lage an Stellen, die von gegenüberliegenden Seitenrändern einer Öffnung, die von gegenüberliegenden inneren Seitenrändern der oberen Lage begrenzt wird, geringfügig nach außen beabstandet angebracht sind.

Bei der vorstehend beschriebenen Wegwerfwindel nach dem Stand der Technik sind die elastischen Elemente nicht in umhüllter Form, d.h. also in offenliegender Form an der Unterseite der oberen Lage an Stellen, die geringfügig von den jeweiligen inneren Seitenrändern der oberen Lage, die die einander gegenüberliegenden Seitenränder einer Öffnung bilden, nach außen beabstandet sind, angebracht, wohl weil es für herkömmliche Geräte für die Massenproduktion von Wegwerfwindeln mit hoher Geschwindigkeit praktisch schwierig ist, die elastischen Elemente mittels Klebstoffes an die inneren Seitenränder der oberen Lage in ordentlicher Anordnung aufzukleben. Auch wird der Klebstoff, wenn ein derartiger Klebevorgang versucht wird, teilweise nach innen zu ausquellen und bei der Berührung mit der Haut des Trägers ein unangenehmes Gefühl verursachen. Dieses Ausquellen des Klebstoffes könnte zwar durch Umhüllen der elastischen Elemente mit den inneren Seitenrändern der oberen Lage vermieden werden, ein derartiges Umhüllen der elastischen Elemente erfordert jedoch eine weitere Bearbeitung der inneren Seitenränder, die die Seitenränder der Öffnung bilden, und auch nach der Durchführung einer solchen Bearbeitung ist es mit herkömmlichen Geräten schwierig, ein rasches Verkleben und Umhüllen der elastischen Elemente entlang der so weiterbearbeiteten inneren Seitenränder zu erzielen.

Hier sei angemerkt, daß bei Befestigung der elastischen Elemente an Stellen der oberen Lage, die von den inneren Seitenrändern der oberen Lage nach außen zu beabstandet sind und nicht von den inneren Seitenrändern umhüllt sind, diese inneren Seitenränder nicht in der Lage sind, fein geraffte Fältchen zu bilden. Im Hinblick auf die Tatsache, daß diese inneren Seitenränder mit der Haut des Trägers in Berührung stehen sollen und wirksame Dichtungen bilden sollen, wird die angestrebte Wirkung, das Austreten von Exkrementen, insbesondere flüssigen Exkrementen zu verhindern, durch das Fehlen von Fältchen schwer beeinträchtigt. Eine ähnliche Wegwerfwindel ist aus der EP-A-0 421 473 bekannt.

Demgemäß ist es eine hauptsächliche Aufgabe der Erfindung, Wegwerfwindeln aufzuzeigen, bei welchen das Problem vermieden werden kann, indem zwei separat ausgebildete dehnbare Klappen beiderseits der in der inneren oberen Lage gebildeten Öffnung vorgesehen sind.

### BESCHREIBUNG DER ERFINDUNG

Eine erfindungsgemäße Wegwerfwindel umfaßt einen integralen Schichtkörper aus einer flüssigkeitsdurchlässigen ersten oberen Lage, eine flüssigkeitsundurchlässige untere Lage, einen zwischen der ersten oberen Lage und der unteren Lage angeordneten flüssigkeitsabsorbierenden Kern, und eine flüssigkeitsabweisende zweite obere Lage, die über der ersten oberen Lage liegt, wobei die zweite obere Lage im wesentlichen in ihrem zentralen Bereich mit einer ersten Öffnung versehen ist, deren Abmessung in Längsrichtung größer ist als in Querrichtung, und entlang ihrem äußeren Umfang auf die erste obere Lage geklebt ist, und wobei unmittelbar an gegenüberliegenden Seitenrändern der ersten Öffnung in Längsrichtung zu dieser verlaufend elastische Elemente vorgesehen sind, die in Längsrichtung der ersten Öffnung dehnbar sind, wobei die Verbesserung umfaßt,
daß an beiden Seiten der ersten Öffnung zwei Klappen vorgesehen sind, die oberhalb des Kernes von den Unterseiten der jeweiligen Seitenabschnitte der zweiten oberen Lage nach innen verlaufen, so daß zwischen gegenüberliegenden inneren Seitenrändern eine zweite Öffnung gebildet ist, in Längsrichtung gegenüberliegende Enden der jeweiligen Klappe sich unter die zweite obere Lage erstrecken, die inneren Seitenränder dieser Klappen die elastischen Elemente umhüllen, und die äußeren Seitenbereiche dieser Klappen an der Unterseite der zweiten oberen Lage verklebt sind, die über der jeweiligen Klappe liegt.

Vorzugsweise sind die äußeren Seitenabschnitte der Klappen mit Klebstoff an die Unterseite der Bereiche der zweiten oberen Lage geklebt, die über den äußeren Seitenabschnitten liegen, so daß die jeweiligen inneren Seitenränder der ersten Öffnung frei bleiben.

Bei der Benutzung der Windel mit vorstehend beschriebenem Aufbau gemäß der Lehre der Erfindung werden die gegenüberliegenden inneren Seitenabschnitte der zweiten oberen Lage unter der Kontraktion der jeweiligen elastischen Elemente von der ersten oberen Lage abgehoben und bilden feine Fältchen, die dicht an der Haut des Trägers anliegen, so daß die Exkremente durch die von den inneren Seitenrändern der zweiten Lage gebildete Öffnung in zwischen der ersten und zweiten oberen Lage ausgebildete Taschen fließen und in der Windel gehalten werden.

Die erfindungsgemäße Windel hat insoweit einen einzigartigen Aufbau, als daß getrennt von der zweiten oberen Lage zwei Klappen vorgesehen sind, die die gegenüberliegenden Seitenränder der zweiten Öffnung bilden. Die elastischen Elemente sind jeweils von den inneren Seitenrändern umhüllt und verleihen diesen inneren Seitenrändern elastische Dehnbarkeit. Die äußeren Seitenbereiche der jeweiligen Klappen sind mit der Unterseite der zweiten oberen Lage an deren gegenüberliegenden Seitenbereichen verklebt. Durch diesen Aufbau können einerseits die elastischen Elemente an den gewünschten Stellen angebracht werden, wie etwa den inneren Seitenrändern der Öffnung, ohne daß dabei Klebstoff ausquillt oder offengelegt wird, und andererseits können an den inneren Seitenrändern der jeweiligen Klappen feine Fältchen gebildet werden. Durch diese Fältchen passen sich die inneren Seitenränder der jeweiligen Klappen an die Haut des Trägers an und die Dämmwirkung gegen den Austritt von Exkrementen ist verbessert.

Als weiteres Merkmal der Erfindung sind die außenseitigen Bereiche der Klappen jeweils mit Klebstoff mit der Unterseite der zweiten oberen Lage in den die außenseitigen Bereiche jeweils überdeckenden Abschnitten derselben verklebt, wobei die inneren Seitenränder der Abschnitte der zweiten oberen Lage frei bleiben. Durch diesen Aufbau können möglicherweise über die inneren Seitenränder der Klappen fließende Exkremente von den zwischen den inneren Seitenrändern der ersten Öffnung, die von der oberen Lage gebildet werden, und den Klappen gebildeten schlitzartigen Taschen zurückgehalten werden.

Dieses Merkmal sowie weitere Merkmale und Vorteile der Erfindung sind anhand der folgenden Beschreibung unter Bezug auf die beiliegende Zeichnung leichter verständlich.

### KURZE BESCHREIBUNG DER FIGUREN

- Fig. 1: zeigt eine isometrische Ansicht der Innenseite eines Beispiels einer gemäß der Erfindung aufgebauten Wegwerfwindel;
- Fig. 2: zeigt eine isometrische Teilschnittdarstellung entlang der Linie 2 - 2 in Fig. 1;
- Fig. 3: zeigt eine Schnittdarstellung entlang der Linie 2 - 2 in Fig. 1; und
- Fig. 4: zeigt eine Draufsicht auf die Innenseite der entfalteten Windel.

### BEVORZUGTE AUSFÜHRUNGSFORMEN DER ERFINDUNG

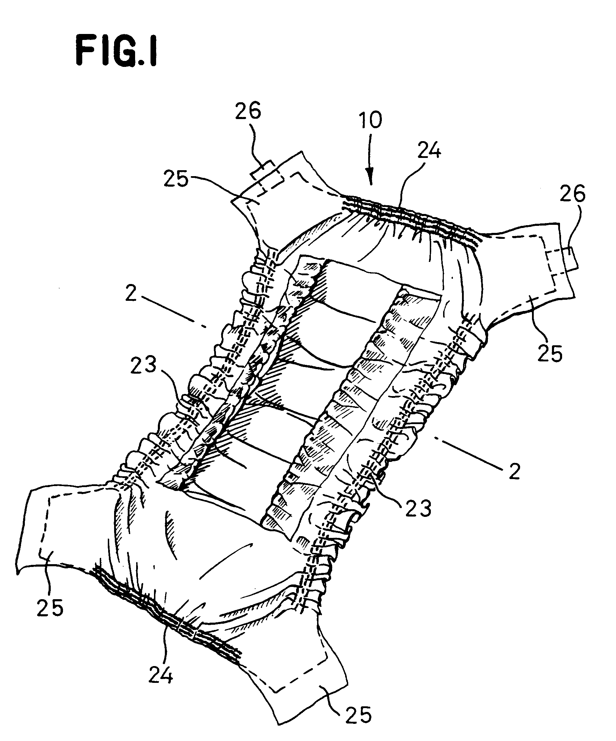

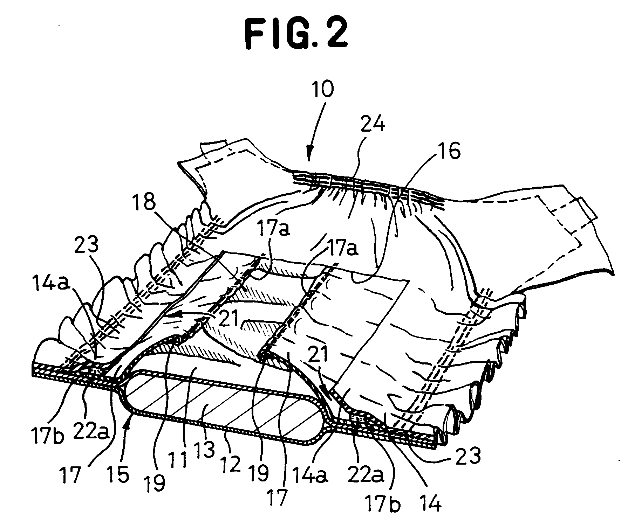

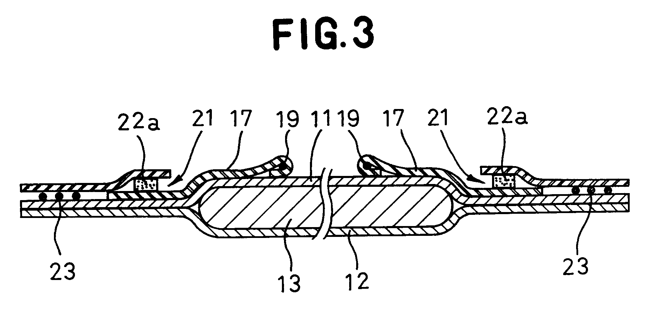

Wie in Fig. 1 bis 3 gezeigt, umfaßt eine Windel 10 einen Schichtkörper 15, der wiederum eine flüssigkeitsdurchlässige erste obere Lage 11, eine flüssigkeitsundurchlässige untere Lage 12, einen zwischen diesen Lagen angeordneten flüssigkeitsabsorbierenden Kern 13 und eine flüssigkeitsabweisende zweite obere Lage 14, die über der ersten oberen Lage 11 liegt. Im Mittelbereich der zweiten oberen Lage 14 ist eine erste Öffnung 16 ausgebildet, deren Abmessung in Längsrichtung größer ist als in Querrichtung. Die Öffnung 16 kann so positioniert sein, daß sie wenigstens einen Schrittbereich einnimmt.

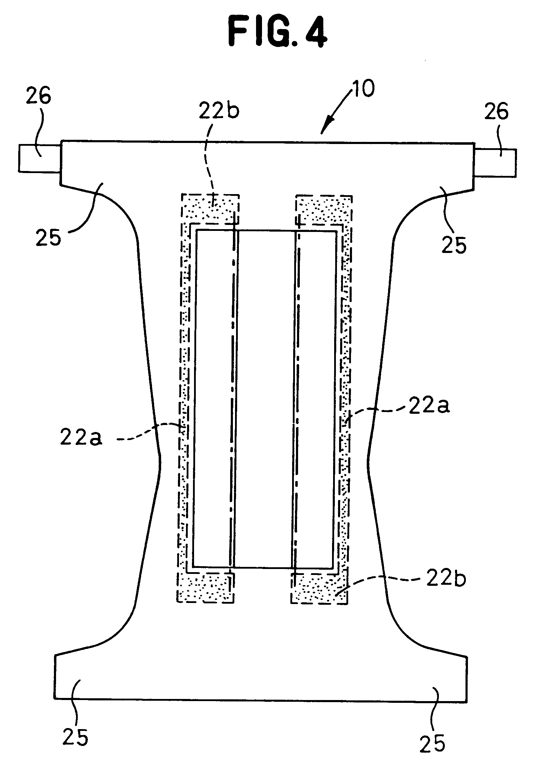

Zwei relativ schmale flüssigkeitsabweisende Klappen 17 erstrecken sich über dem Kern 13 von den Unterseiten von jeweiligen Seitenbereichen 14a der zweiten oberen Lage nach innen, so daß einander gegenüberliegende innere Seitenränder 17a der jeweiligen Klappen 17 zwischen sich eine zweite Öffnung 18 bilden. In Längsrichtung gegenüberliegend angeordnete Enden der Klappen 17 verlaufen jeweils unter die zweite obere Lage 14. Die innere Seitenkante 17a der Klappen 17 ist jeweils mit einem in Längsrichtung dehnbaren elastischen Element 19 versehen, das mit diesen verklebt und von diesen umhüllt ist. Ein äußerer Endbereich 17b der Klappen 17 ist jeweils mit dem Seitenbereich 14a der zweiten oberen Lage 14, der über dem äußeren Endbereich 17b liegt, durch eine Klebstofflinie 22a verklebt, wobei der innere Seitenrand der zweiten oberen Lage 14 oder die erste Öffnung 16 frei bleibt, so daß eine schlitzförmige Tasche 21 zwischen dem jeweiligen inneren Seitenrand der ersten Öffnung 16, der von der zweiten oberen Lage 14 gebildet wird, und der zugehörigen Klappe 17 ausgebildet ist. Die in Längsrichtung einander gegenüberliegenden Enden der Klappen 17 sind jeweils mit Klebstoff 22b mit der zweiten oberen Lage 14 verklebt (siehe Fig. 4).

Zwischen Bereichen der ersten oberen Lage 11 und der unteren Lage 12, die beiderseits des Kerns 13 nach außen verlaufen, einerseits und den zugehörigen Seitenbereichen 14a der zweiten oberen Lage 14 andererseits sind mehrere elastische Elemente 23 vorgesehen, die in Längsrichtung dehnbar sind und sich so jeweils dem Bein des Trägers anpassen. In ähnlicher Weise sind an den in Längsrichtung gegenüberliegenden Enden zwischen den Lagen 11, 12 und der Lage 14 mehrere elastische Elemente 24 vorgesehen, die in Querrichtung dehnbar sind und sich so der Taille des Trägers anpassen.

Die erste obere Lage 11 kann aus Vliesstoff, poröser Kunststoffolie oder ähnlichem bestehen. Die untere Lage 12 kann aus Kunststoffolie, einem Schichtstoff aus der Kunststoffolie und einem Vliesstoff oder ähnlichem bestehen. Der Kern 13 kann aus Faserpulpe, gemischt mit einem hochabsorbierenden Polymerpulver oder ähnlichem bestehen. Die zweite obere Lage und die Klappen 17 sind jeweils vorzugsweise aus flüssigkeitsundurchlässigem, aber luftdurchlässigem Vliesstoff hergestellt. Der hier verwendete Ausdruck "flüssigkeitsabweisend" ist so zu verstehen, daß die wasserabweisende Eigenschaft der zweiten oberen Lage ausreicht, um das unmittelbare Durchdringen der zweiten oberen Lage durch flüssige Exkremente während der Benutzung der Windel zu verhindern.

Wie aus Fig. 1 ersichtlich ist, sind an der Windel 10 an den gegenüberliegenden Enden der vorderen und hinteren Taillenlinie nach außen verlaufende Laschen 25 angeordnet und die Laschen 25 der hinteren Taillenlinie mit Klebebändern 26 versehen, so daß die freien Enden dieser Klebebänder 26 vorübergehend an die untere Lage 12 im vorderen Abschnitt der Windel angeklebt werden können, um die Windel dem Träger anzulegen.

## Patentansprüche

1. Wegwerfwindel (10), umfassend einen integralen Schichtkörper (15) mit
- einer flüssigkeitsdurchlässigen ersten oberen Lage (11), einer flüssigkeitsundurchlässigen unteren Lage (12),
- einem zwischen der ersten oberen Lage und der unteren Lage liegenden flüssigkeitsabsorbierenden Kern (13),
- und einer flüssigkeitsabweisenden zweiten oberen Lage (14), die über der ersten oberen Lage liegt (11),
- wobei die zweite obere Lage (14) im wesentlichen in ihrem Mittelbereich mit einer ersten Öffnung (16) versehen ist, deren Abmessung in Längsrichtung größer ist als in Querrichtung und die entlang ihrem äußeren Umfang auf die erste obere Lage (11) geklebt ist,
- und wobei unmittelbar an gegenüberliegenden Seitenrändern der ersten Öffnung (16) in Längsrichtung zu dieser verlaufend elastische Elemente (19) vorgesehen sind, die in Längsrichtung der ersten Öffnung dehnbar sind, wobei ferner an beiden Seiten der ersten Öffnung zwei Klappen (17) vorgesehen sind, die oberhalb des Kernes (13) von den Unterseiten der jeweiligen Seitenabschnitte (14a) der zweiten oberen Lage (14) nach innen verlaufen, so daß gegenüberliegende innere Seitenränder (17a) zwischen sich eine zweite Öffnung (18) bilden, in Längsrichtung gegenüberliegende Enden der Klappen (17) sich jeweils unter die zweite obere Lage (14) erstrecken, die inneren Seitenränder (17a) dieser Klappen (17) die elastischen Elemente (19) umhüllen, und die äußeren Seitenbereiche (17b) dieser Klappen (17) an der Unterseite der zweiten oberen Lage (14) verklebt sind, die über der jeweiligen Klappe (17) liegt.

2. Wegwerfwindel nach Anspruch 1,
dadurch gekennzeichnet, daß die äußeren Seitenbereiche der Klappen jeweils mit Klebstoff mit der Unterseite der Bereiche der zweiten oberen Lage verklebt sind, die über den äußeren Seitenbereichen liegen, wobei die jeweiligen inneren Seitenränder der ersten Öffnung frei bleiben.

## Claims

1. Disposable nappy (10) comprising an integral layered body (15) with
- a first upper layer (11) that is permeable by liquid, a non-porous lower layer (12),
- an absorbent centre (13), lying between the first upper layer and the lower layer,
- and a liquid-repellent second upper layer (14) lying above the first upper layer (11),
- where the second upper layer (14) is provided substantially in its middle area with a first opening (16), the measurement of which is greater lengthwise than crosswise and which is glued along its outer perimeter to the first upper layer (11),
- and where directly on opposite side edges of the first opening (16) elastic components (19) are provided which run lengthwise along the first opening and are stretchable lengthwise along the first opening, where, in addition, on both sides of the first opening two flaps (17) are provided, which run inwards above the centre (13) from the undersides of each side section (14a) of the second upper layer (14), in such a way that opposite inner side edges (17a) form between themselves a second opening (18), lengthwise opposite ends of the flaps (17) each extend under the second upper layer (14), the inner side edges (17a) of these flaps (17) surround the elastic components (19), and the outer side areas (17b) of these flaps (17) are glued on to the underside of the second upper layer (14), which lies above each flap (17).
glued on to the underside of the second upper layer (14), which lies above each flap (17).

2. Disposable nappy according to claim 1,
characterised in that the external lateral regions of the flaps are respectively glued with adhesive to the underside of the regions of the second upper layer which lie above the external lateral regions, the respective internal lateral edges of the first opening remaining free.

## Revendications

1. Lange jetable (10), comprenant une structure lamifiée intégrale (15) comportant :
- une première couche supérieure, perméable aux liquides (11), une couche inférieure imperméable aux liquides (12),
- un corps central (13) absorbant les liquides, disposé entre la première couche supérieure et la couche inférieure,
- et une seconde couche supérieure hydrophobe (14), qui se trouve au-dessus de la première couche supérieure (11),
- lange dans lequel la seconde couche supérieure (14) est pourvue, essentiellement dans sa zone centrale, d'une première ouverture (16) dont l'étendue est plus grande dans le sens longitudinal que dans le sens transversal, et est collée, le long de son pourtour extérieur, à la première couche supérieure (11),
- et où des éléments élastiques (19), étirables dans le sens de la longueur de la première ouverture, sont prévus immédiatement sur des bords latéraux opposés de la première ouverture (16), dans le sens de la longueur de cette dernière, et où, en outre,
- il est prévu, de part et d'autre de la première ouverture, deux pattes (17) qui partent vers l'intérieur, par dessus le corps central (13), venant du dessous de chacune des portions latérales (14a) de la seconde couche supérieure (14), de sorte que des bords latéraux intérieurs opposés (17a) forment, entre eux, une seconde ouverture (18), que des extrémités des pattes (17), opposées dans le sens de la longueur, passent sous la seconde couche supérieure (14), que les bords latéraux intérieurs (17a) de ces pattes (17) enveloppent les éléments élastiques (19), et que les bords latéraux extérieurs (17b) de ces pattes (17) sont collés à la face inférieure de la seconde couche supérieure (14) qui se trouve au-dessus de la patte respective (17).

2. Lange jetable selon la revendication 1, caractérisé en ce que les zones latérales extérieures des pattes sont respectivement collées, avec de la colle, à la face inférieure des zones de la seconde couche supérieure qui se trouvent au-dessus des zones latérales extérieures, les bords latéraux intérieurs respectifs de la première ouverture restant libres.
